# EUROPEAN PATENT APPLICATION

(11) **EP 4 548 921 A1**
(43) Date of publication of application: **07.05.2025**
(21) Application number: 23207156.3
(22) Date of filing: 31.10.2023
(51) Int. Cl.: A61K 31/496, A61K 31/5025, A61P 21/00

(54) **COMPOUNDS FOR THE TREATMENT OF HUMAN SARCOPENIA**

(71) Applicant: ScandiEdge Therapeutics AB, 121 36 Johanneshov (SE)
(72) Inventor: MARDINOGLU, Adil, 412 60 Göteborg (SE); BORÉN, Jan, 414 67 Göteborg (SE); UHLÉN, Mathias, 181 90 Lidingö (SE)
(74) Representative: Kransell & Wennborg KB

(57) **Abstract**

There is provided a compound, a pharmaceutically acceptable salt or a prodrug thereof for use in a method of treatment of human sarcopenia, wherein the compound is Mitapivat or TEPP46.

## Description

### TECHNICAL FIELD

The present disclosure relates to the field of treatment of human sarcopenia.

### BACKGROUND

Sarcopenia has become prevalent among elderly subjects. It is characterized by gradual loss of skeletal muscle mass, strength, and function. Physiologically, skeletal muscle generates force for essential functions such as swallowing, respiration, and movement. On a molecular level, skeletal muscles maintain cellular homeostasis, playing roles in glucose and lipid metabolism, thermogenesis, and inflammation. [1],[2] Additionally, skeletal muscle acts as a storage source for amino acids and serves as an energy source during starvation. [2],[3] It has been reported that sufficient muscle mass is important to prevent obesity and type 2 diabetes. [3] Therefore, sarcopenia affects the quality of life by lowering the ability to perform daily tasks and reducing the protection of the vital organs in the body.

Sarcopenia is characterized by the lack of myogenic cell differentiation. Myogenic cell differentiation progresses in several stages, which is regulated by muscle-specific transcription factors (TFs). When adult muscle satellite cells express the transcription factors myoblast determination protein 1 (MYOD) and/or myogenic factor 5 (MYF5), myoblasts can further progress into differentiation towards myotubes. During differentiation, myoblasts fuse into myotubes by expressing transcription factor myogenin (MYOG). [4] The activation and expression of these TFs tightly interact with the biological systems including key metabolites, enzymes, and biological pathways. So far, there are no therapeutics for the effective treatment of sarcopenia in clinical practice. Therefore, there is a need to develop effective treatments and drugs for the treatment of muscle sarcopenia.

### SUMMARY

Previous studies have shown that glucose concentration can regulate skeletal muscle differentiation through 5' adenosine monophosphate-activated protein kinase (AMPK) or protein kinase B (AKT) signalling pathways, [5],[6] implicating the importance of glycolysis and gluconeogenesis in the muscle cell differentiation from myoblasts to myotubes.

In the glycolysis pathway, pyruvate kinase (PK) is a pivotal late-limiting enzyme of glycolysis, which catalyses its substrate phosphoenolpyruvate (PEP) to pyruvate. [7] Pyruvate kinase M1 (PKM1) and M2 (PKM2) are two isoforms that are products of alternative splicing of the PKM gene. [8] PKM2 has a dimeric form and a tetrameric form; the latter is the active form of PKM2. PKM2 is allosterically activated by glycolytic intermediate metabolite fructose-1,6-bisphosphate (FBP). [9] Previous studies have shown that PKM2 promotes muscle progenitor cell (MPC) proliferation, [10] and activated PKM2 can stabilize myogenic factor MyoD and subsequently enhance myogenesis. [11]

The present inventors hypothesized that PK activation could lead to favourable outcomes in the treatment of sarcopenia. By examining the potential dysregulation in the glycolysis pathway in muscle tissues from sarcopenia patients, an *in vitro* sarcopenia model was established. The *in vitro* sarcopenia model uses C2C12 mouse myoblasts treated with N-acetyl-D-sphingosine (C2-ceramide) - a compound known to inhibit myogenic differentiation and induce skeletal muscle senescence. [12] It was shown, using the in vitro sarcopenia model, that activation of PK using Mitapivat (MTPV) and TEPP46 could be used in the treatment of sarcopenia. The therapeutic effects of these compounds were further determined by generating RNA-sequencing (RNA-seq) data as well as performing immunofluorescence staining. The mechanisms of these compounds were subsequently demonstrated by leveraging gene co-expression network analysis.

Accordingly, the present disclosure provides a compound or a pharmaceutically acceptable salt or a prodrug thereof for use in a method of treatment of human sarcopenia, wherein the compound is selected from Mitapivat (MTPV) and TEPP46.

The present inventors found that MTPV and TEPP46 can alleviate the sarcopenia phenotype based on the *in vitro* sarcopenia model by activating the myogenesis pathway and modulating the mitochondrial dysfunction. Therefore, treatment with MTPV and TEPP46 could be used as effective therapeutic alternatives for the treatment of sarcopenia patients.

### BRIEF DESCRIPTION OF THE DRAWINGS

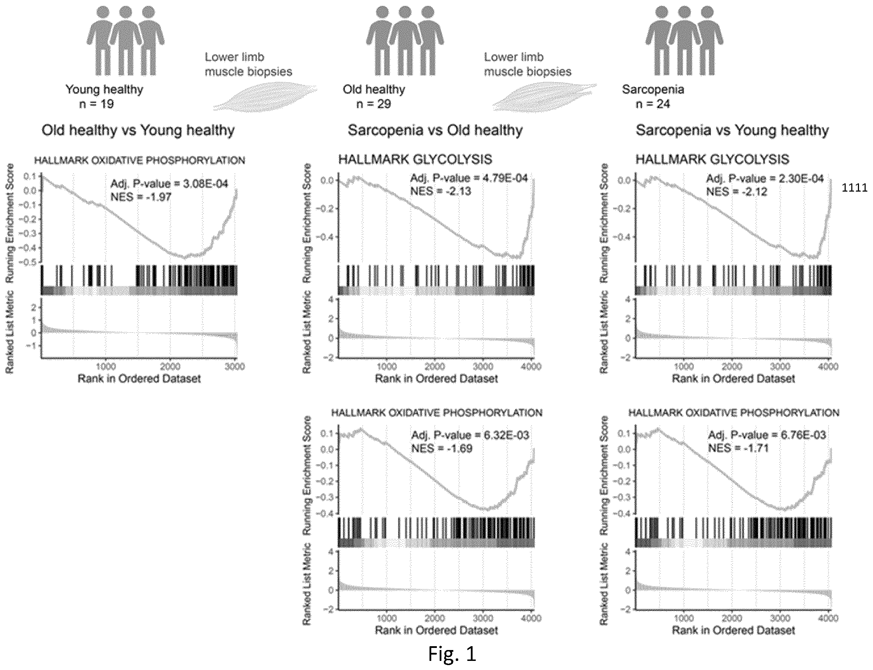

Fig 1 shows that sarcopenia is characterized by the down-regulation of glycolysis and oxidative phosphorylation in human skeletal muscle. The aged human skeletal muscle cohort (GSE167186) consists of 72 biopsies collected from human lower limb muscle tissues (young healthy, n = 19; old healthy, n = 29; sarcopenia, n = 24). These three groups were compared in a pairwise manner for differential gene expression analysis and the MSigDB hallmark gene sets were evaluated for their significance based on GSEA. The two most significant pathways (for old healthy versus young healthy, only the significant one) are shown.

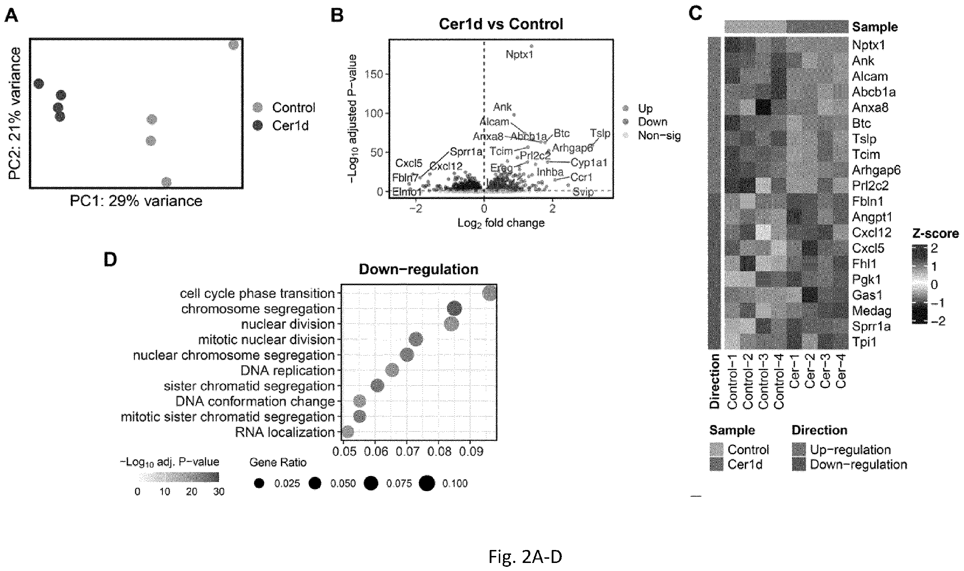

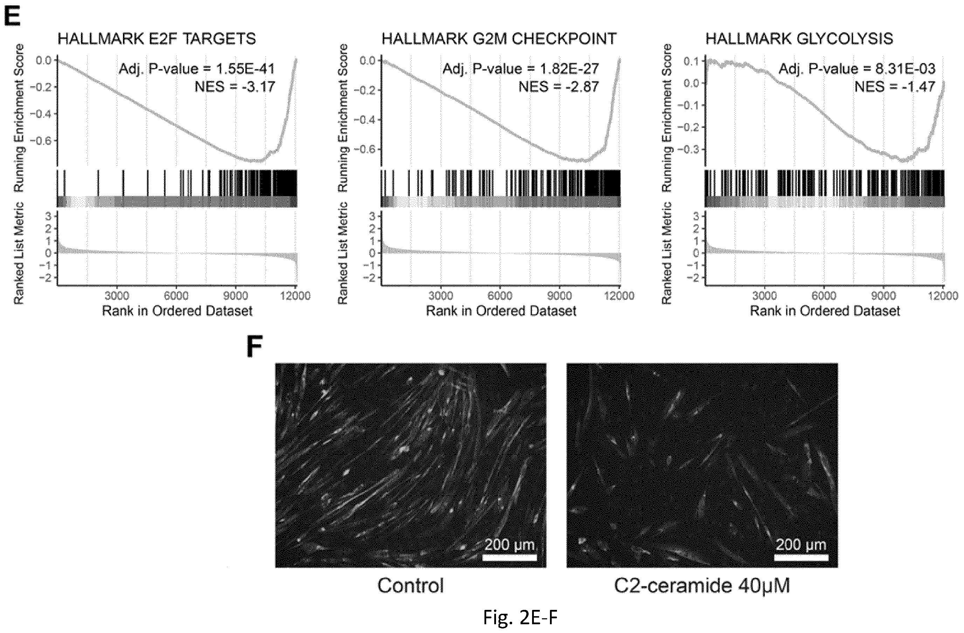

Fig 2 shows an *in vitro* sarcopenia model, established by treating C2C12 cells with ceramide. (A) PCA plot shows the sample distribution of the disease model (Cerid; n = 4) and untreated C2C12 mouse myoblasts (Control; n = 4). (B) Volcano plot shows the genes that are differentially expressed between Cerid and Control samples. (C) Heatmap shows the top 10 up-regulated and top 10 down-regulated genes when comparing the Cerid to Control samples (D) Ten top-ranked significant GO terms associated with up- or down-regulated DEGs in Cerid compared to Control are presented. (E) Most representative hallmark gene sets enriched in Cerid compared to Control are evaluated by GSEA. (F) Immunofluorescence staining of Myosin-3 (MYH3) is presented in Cerid and Control samples.

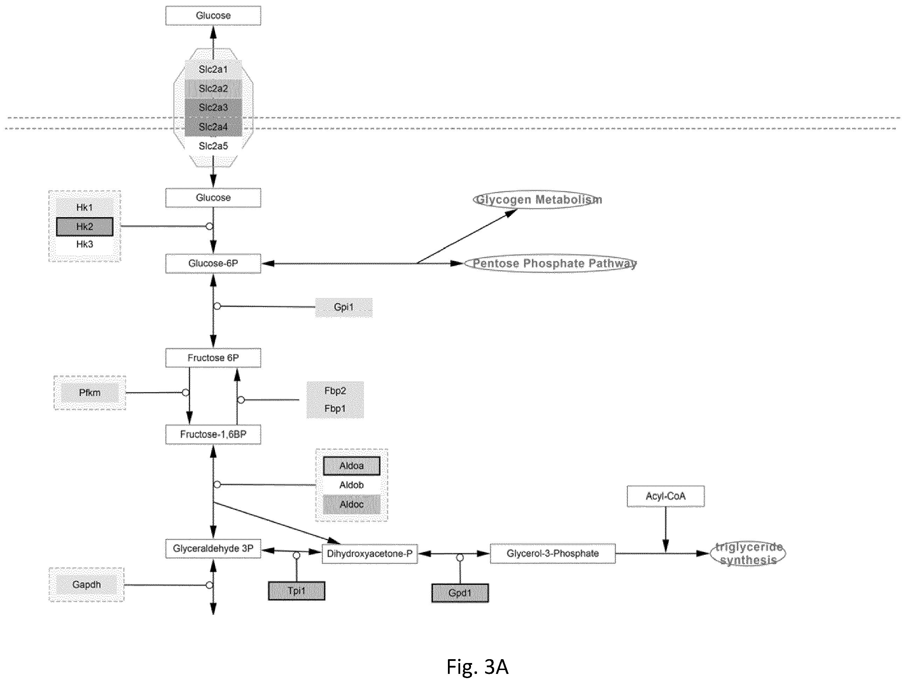

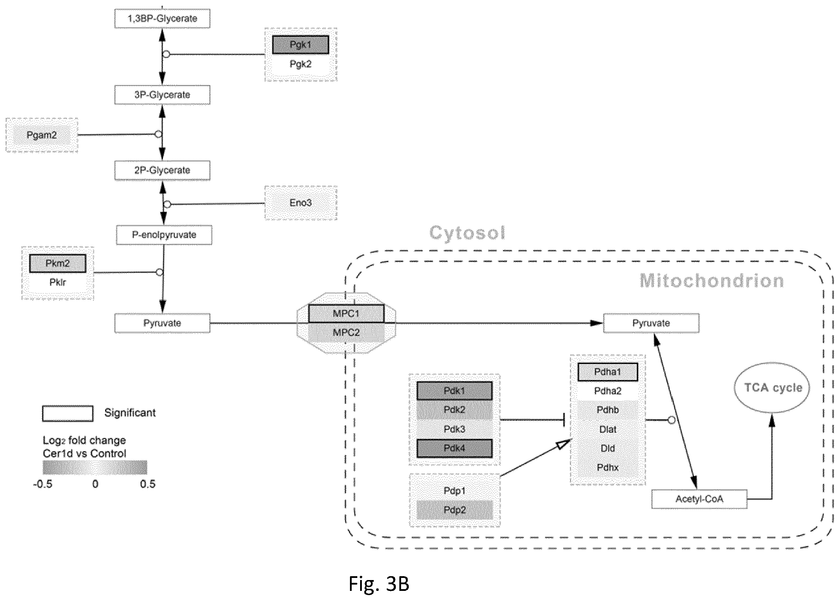

Fig 3A-B shows that the glycolysis pathway is down-regulated in the in vitro sarcopenia model. Visualization of the glycolysis pathway (Mus musculus) based on WP3586 in the WikiPathways database. Genes are colored by log₂ fold change in Cerid compared to Control, with significant DEGs highlighted by a black border.

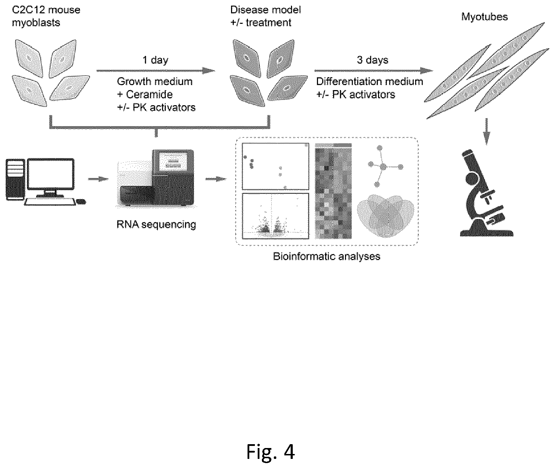

Fig 4 shows the study design for in vitro experiments. The C2C12 mouse myoblast cells were treated by ceramide with growth media for one day to build a sarcopenia disease model or by ceramide and one of the two PK activators with growth media for one day to investigate the effect of the treatment (both n = 4). Disease models with/without treatments were cultured with myotube differentiation media with the same PK activators for three days for immunofluorescence staining. The untreated myoblast cells and the disease models with/without treatment of PK activators were analysed by performing integrative transcriptomics and network analysis.

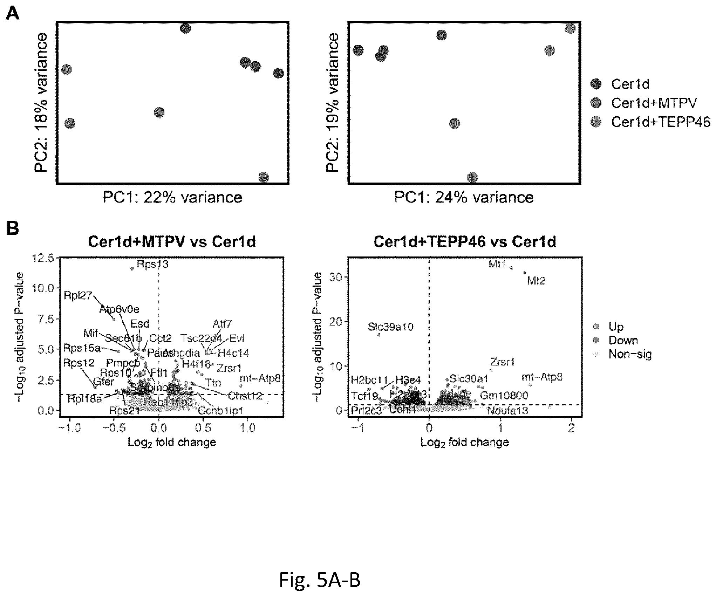

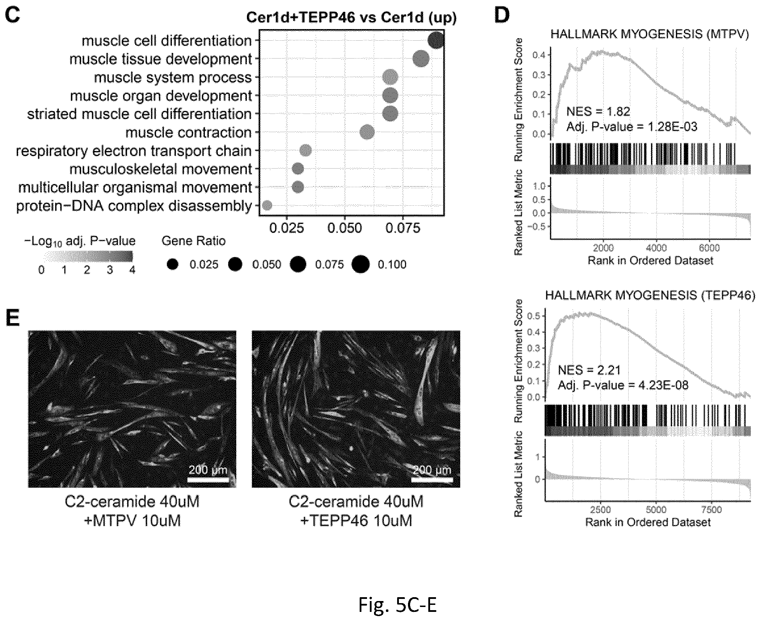

Fig 5 shows that the PK activators induce muscle cell differentiation using the *in vitro* sarcopenia model. (A) PCA plots show the sample distribution between disease models Cer1d (n = 4) and Cend treated with MTPV (n = 4; left) or TEPP46 (n = 4; right). (B) Volcano plot shows the genes that are differentially expressed between Cend + MTPV compared to Cend (left) or Cer1d+TEPP46 compared to Cer1d (right). (C) The 10 top-ranked significant GO terms associated with up-regulated DEGs in Cer1d+TEPP46 compared to Cer1d are presented. (D) The enrichment of the hallmark gene set myogenesis in Cerid+MTPV compared to Cerid (upper) or Cer1d+TEPP46 compared to Cend (lower) by GSEA is shown. (E) Immunofluorescence staining of Myosin-3 (MYH3) in myotubes treated by MTPV or TEPP46 is presented.

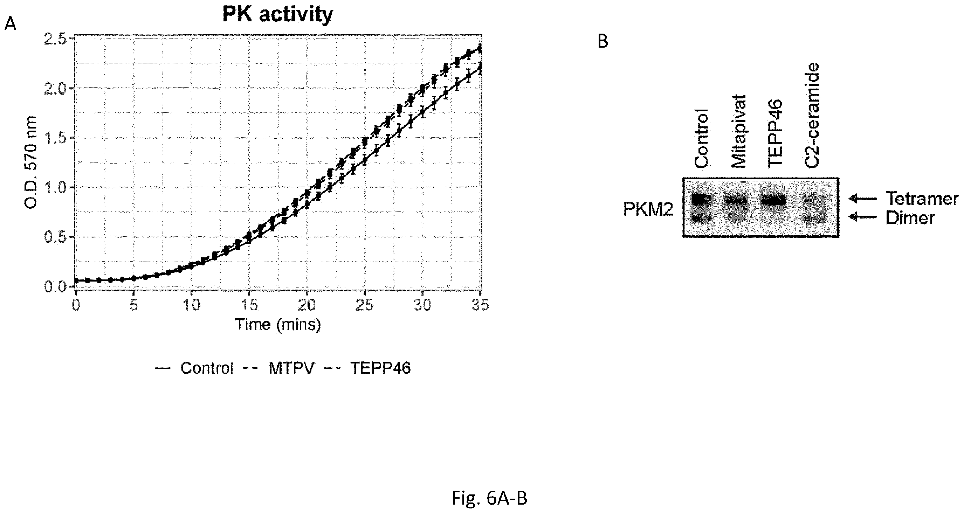

Fig 6 shows that MTPV and TEPP46 activate the PK tetramer. (A) Pyruvate kinase activity assays in cell lysates treated with MTPV and TEPP46 for 1 day (n = 4). (B) Cross-linking was performed on C2C12 cells treated with MTPV and TEPP46 for 1 day with EGS (1 mM, 15 min) and 1% PFA (10 min). The expression of PKM2 dimer and tetramer is shown after treatment with MTPV and TEPP46.

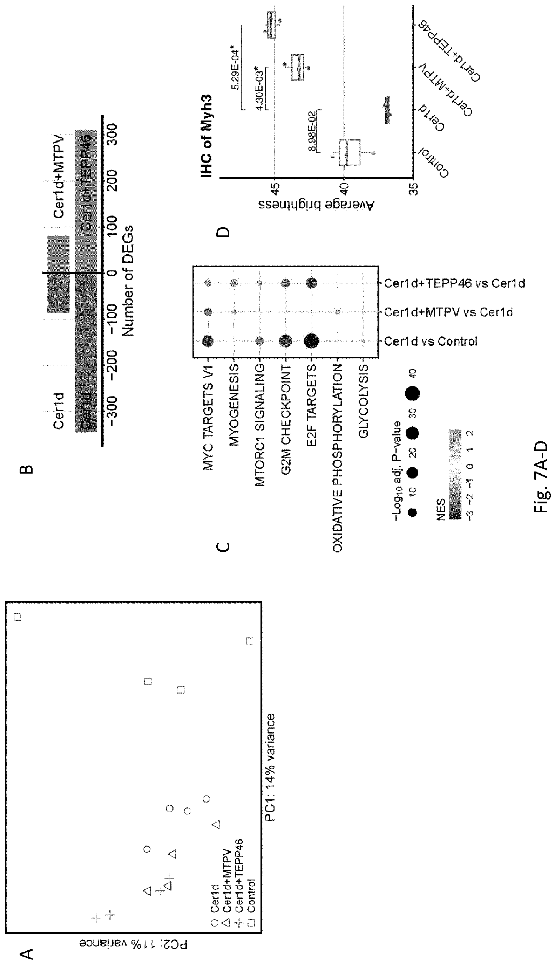

Fig 7 shows that the PK activators show distinct efficacy in the *in vitro* sarcopenia model. (A) PCA plot shows sample distribution of all the 16 analysed RNA-seq samples. (B) Bar plot shows the number of DEGs comparing treatments Cer1d+MTPV/TEPP46 to Cend (C) The enrichment of the seven representative hallmark gene sets in Cend compared to Control and in Cer1d plus the treatments with MTPV and TEPP46 compared to Cer1d are presented. (D) Average brightness of MYH3 quantified by ImageJ based on immunofluorescence staining figures. For each group, n = 3 figures were taken at random areas. Statistical differences were evaluated by Wilcoxon rank-sum test (non-normal distribution) or t-test (normal distribution). Data normality was checked by Shapiro-Wilk test. * p < 0.05.

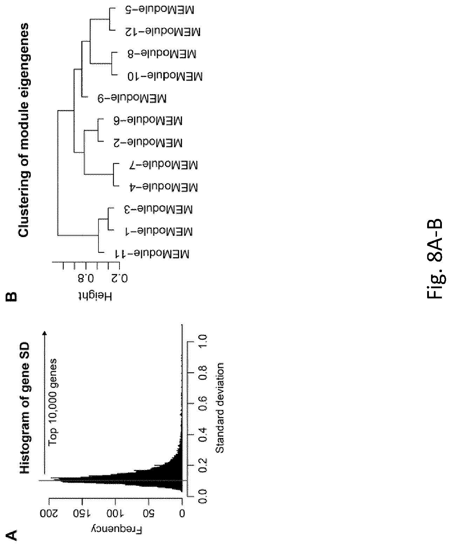

Fig 8 shows the gene co-expression analysis based on cell line RNA-seq data. (A) Histogram shows the distribution of the standard deviation of the 14,661 genes. The red vertical line indicates the threshold for selecting the top 10,000 genes based on the standard deviation. (B) Dendrogram shows the hierarchical clustering of the WGCNA modules based on the module eigengenes.

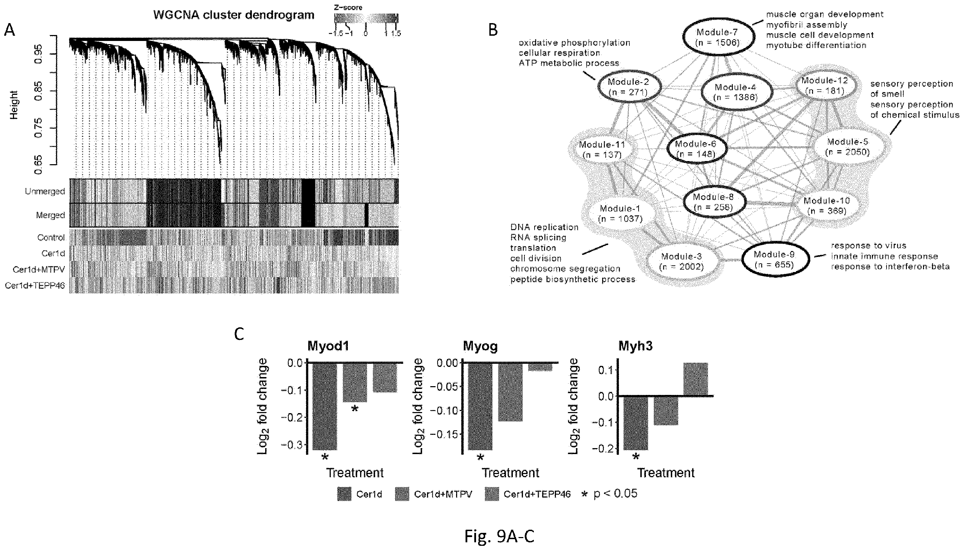

Fig 9 shows the gene co-expression network analysis that identified a subnetwork controlling muscle cell differentiation based on *in vitro* sarcopenia cell line models. (A) Dendrogram of hierarchical clustering of 10,000 analysed genes in WGCNA is shown (Upper). The lower part shows the relative expression of the 10,000 genes in all samples. Expression was z-normalized per gene and the z-scores were averaged per sample group. (B) Network visualization of the WGCNA modules with the associated GO terms annotated. Edge width is calculated based on the average adjacency between all the gene pairs in the two connected co-expression modules. The number of genes in each module is shown in round brackets. (C) The log₂ fold change of three markers for sarcopenia is shown in the disease model Cerid compared to Control (Cerid vs. Control) and cells treated with the PK activators in the disease model (Cerid+activator) compared to Cerid. *p < 0.05 is shown as significant.

### DETAILED DESCRIPTION

In this disclosure, the inventors identified glycolysis as the most dysregulated pathway based on the transcriptomics data analysis of human sarcopenia patients compared to healthy old and young subjects. An in vitro sarcopenia cell line model hallmarked by down-regulation of cell cycle and glycolysis using C2C12 mouse myoblasts treated with C2-ceramide was established. This model was then used to study the effect of MTPV and TEPP46 in the treatment of sarcopenia. Treating the sarcopenia model with MTPV and TEPP46 showed that these compounds reactivated the muscle cell differentiation and resulted in the differentiation of myoblasts into myotubes. Moreover, it was demonstrated, by gene co-expression network analysis and transcription factor analysis, that the dysregulation of key TFs regulating muscle cell differentiation could be alleviated by treatment with MTPV (formula I) and TEPP46 (formula II).

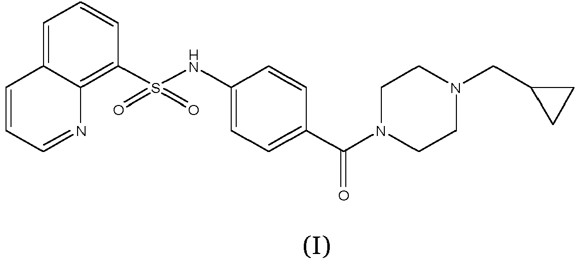

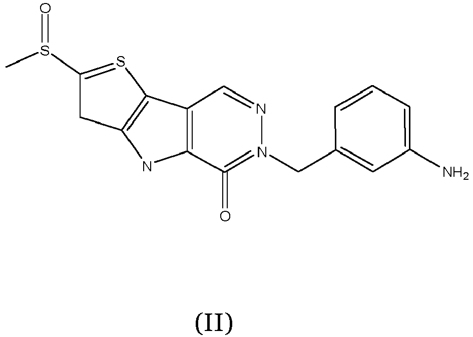

The establishment of *in vivo* animal models for sarcopenia generally requires at least 12 months. [34] Despite the inherent reliability and robustness of the animal models, this time-consuming and high-cost procedure limits animal models as the first step for studying aging-related sarcopenia pathogenesis and for developing new therapeutic strategies. To bypass this limitation, *in vitro* sarcopenia models have been developed, including the treatment of animal muscle cell lines by H₂O₂, sphingolipids (including ceramide), and inflammatory cytokines. [35] The molecular characteristics of C2C12 mouse myoblasts treated with C2-ceramide based on RNA-seq profiling has been shown as well as the similar disease characteristics between the developed *in vitro* model based on ceramide treatment and the human sarcopenia cohort, including the down-regulation of glycolysis and the lack of myotube fusion.

Human sarcopenia is led by impaired muscle regeneration. This occurs due to the decreased self-renewal and differentiating capacity of satellite cells. [36] During muscle regeneration, satellite cells undergo proliferation and generate myoblasts expressing transcription factor MYOD for myogenesis. When myoblasts exit the cell cycle, these cells differentiate into myocytes meanwhile expressing myogenin (MYOG). Myocytes fuse to form myotubes that express myosin heavy chains (MYH). [37] These TFs were identified by the present inventors by co-expression and transcription factor analyses based on the cell line RNA-seq dataset, highlighting the potential role of these TFs in regulating muscle cell differentiation. In the *in vitro* sarcopenia model, the expression of Myod1, Myog and Myh3 (all involved in muscle differentiation) was decreased by C2-ceramide. This means that myoblasts lost their ability to differentiate into myotubes. C2-ceramide also changed the expression of genes that contribute to glycolysis or gluconeogenesis, including HK2, Aldoa, PKM2, and PDK4. Genetic ablation of HK2 inhibits malignant growth, as HK2 functions in autophagy regulation and cell death inhibition when it docks to mitochondria. [38] Aldolase A mRNA and protein expression are highly expressed in differentiating myoblasts, and Aldolase A protein level is highly expressed in regenerating muscle. [39] Elevated PDK4 protein expression induces shrinkage of C2C12 myotubes and muscle atrophy. [40] PKM2 acts as a contributing factor to muscle growth. [41] Also, the proliferation of vascular smooth muscle cells is related to PKM2-dependent glycolysis. [42] As can be seen, many of the molecules involved in glycolysis and gluconeogenesis contribute to muscle differentiation. Therefore, the blockage of myogenesis under the treatment of C2-ceramide C2C12 cells could be due to dysregulated glycolysis and gluconeogenesis pathways.

As a hallmark of sarcopenia, the downregulation of glycolysis was presented in both the *in vitro* sarcopenia model and muscle tissues obtained from the human sarcopenia cohort, highlighting the importance of glycolysis and the potential of this pathway for the development of treatment strategies. Furthermore, significant down-regulation of oxidative phosphorylation was detected in the human sarcopenia cohort. Both glycolysis and oxidative phosphorylation produce adenosine triphosphate (ATP) to supply energy to maintain biological processes in muscle cells. It has been reported that mitochondrial dysfunction, i.e., the impaired ability of mitochondria to produce ATP, is associated with aged muscle and sarcopenia. [43],[44] As a product of glycolysis and a substrate of oxidative phosphorylation, pyruvate plays a pivotal role in modulating ATP generation. Activating PK in muscle cells may rebalance energy homeostasis to maintain myoblast-myotube differentiation and muscle cell regeneration.

C2-ceramide-treated C2C12 myoblasts were treated with the TEPP46 and MTPV. It was found that treatment with TEPP46 and MTPV could change the PK dimer form to the more active tetramer form. TEPP46 also showed a stronger effect in myogenesis than MTPV.

So far, there is no medication being approved by the US Food and Drug Administration. [46],[47] Sarcopenia patients are recommended mostly physical exercise and supplements. Thus, there is a need to develop more effective drugs for the treatment of sarcopenia that can modulate muscle cell regeneration. It was found that MTPV and TEPP46 can alleviate the sarcopenia phenotype based on the *in vitro* sarcopenia model by activating the myogenesis pathway and potentially modulating the mitochondrial dysfunction.

Accordingly, there is provided a compound or a pharmaceutical acceptable salt or a prodrug for use in a method of treatment of human sarcopenia, wherein the compound is selected from mitapivat and TEPP46. Hence, treatment with MTPV and TEPP46 could be used as effective therapeutic alternative for the treatment of sarcopenia patients.

### EXAMPLES

### Examples - Methods

### Materials

The C2C12 myoblast cell line, derived from the leg muscle of a normal adult C3H mouse (91031101), and N-acetyl-D-sphingosine (C2-ceramide, A7191) were purchased from Sigma. TEPP46 (HY-18657) and Mitapivat (HY-12689) were purchased from MedChemExpress. Anti-Myosin/MYH3 (ab124205) was purchased from Abcam. Anti-PKM (D78A4) was purchased from Cell Signaling.

### Cell culture

The C2C12 myoblasts were maintained in modified Eagle's medium (DMEM, D-6429) supplemented with 10% fetal bovine serum (FBS) and 1% penicillin/streptomycin (PS) solution and were incubated in a humidified incubator at 37 °C with 5% CO₂. Totally 3×105 cells were seeded in a 6-well plate per well. The cells were incubated overnight and then treated with 40 µM C2-ceramide and with/without 10 µM MTPV or TEPP46 (PK activators) in the maintained medium for 24 hours. Then the medium was changed to the differentiation medium (DMEM with 2% FBS and 1% PS solution) with/without the same PK activators for three days. As a control, cells were treated with DMSO, and the cells were collected after 1-day treatment for RNA-sequencing.

### Immunofluorescence staining

After three days of differentiation, cells were washed three times with phosphate-buffered saline (PBS) solution and fixed in 4% paraformaldehyde solution at 37 °C. After 10 minutes, cells were washed three times with PBS again and permeabilized with 0.1 % TritonX-100 for 15 minutes at room temperature. The cells were incubated for 1 hour in 2 % bovine serum albumin (BSA) solution for blocking, followed by overnight incubation at 4 °C with anti-Myosin/MYH3 (diluted by 0.1% BSA). Then the primary antibody solution was washed away with PBS, and fluorescent dye-conjugated secondary antibody (diluted by 0.1% BSA) was added to the cells. Cells were incubated without light for 45 minutes at room temperature and then washed three times with 1×PBS-T and mounted with a fluorescence mounting medium containing DAPI (ab104139). With cover glasses on cells, images were taken with ZOETM Fluorescent Cell Imager (Bio-rad). Images were analyzed for the Myosin-3 protein intensity by ImageJ15 (v1.53k) by first converting color images into 8-bit black and white images, followed by the operation "Image - Adjust - Threshold" to highlight the staining area. The minimal threshold was set as 25 to filter out background noise. The average brightness of the staining area was quantified by clicking "Analyze - Measure".

### Western blots

Cells were washed with PBS and lysed with CelLytic M (C2978, Sigma-Aldrich) lysis buffer containing protease inhibitors (11836170001, Roche, Switzerland). After 5 minutes of centrifuging at 12,000 rpm, the supernatants of the lysates were collected. Protein Assay Dye Reagent (5000006, Bio-Rad) was used for the determination of protein concentration. The absorbance of the proteins was measured spectrophotometrically at 595 nm by a microplate reader (Hidex Sense Beta Plus). Protein was separated by Mini-PROTEAN^{®} TGXTM Precast Gels (4561086, Bio-Rad, USA), and was transferred to a Trans-Blot Turbo Mini 0.2 µm PVDF Transfer Packs membrane (1704158, Bio-Rad) by using Trans-Blot^{®} TurboTM Transfer System (BioRad). Then, the membrane was blocked with 5% skim milk for 30 min at 4 °C with gentle rocking, followed by TBST washing. Primary antibodies against PKM and α-Tubulin were added for overnight incubation at 4 °C. Goat anti-rabbit HRP or goat anti-mouse IgG-HRP was used as the secondary antibody to bind with the primary antibody for 30 minutes at 4 °C. The protein bands on the membrane were revealed using enhanced chemiluminescence substrate (WBLUF0500, Merck, USA) and detected with ImageQuant^{™} LAS 500 (29-0050-63, GE).

### Pyruvate Kinase assay

Pyruvate kinase assay kit (ab83432) was purchased for PK activity testing in cell lysates. C2C12 cells were seeded to a 96-well plate with 20,000 cells per well. After overnight incubation, 10 µM PK activators and DMSO were used to treat the cells with the fresh medium as experimental and control groups, respectively. After 24 hours of treatment, PK activities were detected following the manufacturer's protocol.

### Cross-linking

Cells were seeded in a 6-well plate and treated with PK activators. After 24 hours, the medium was discarded, and cells were treated with 1 mM Ethylene glycol bis (succinimidyl succinate) (EGS) for 15 minutes, followed by 10 minutes of 1% Paraformaldehyde (PFA) treatment. Then cells were washed with PBS and collected for western blot.

### RNA preparation and sequencing

Four replicates were designed for each experimental group (control group, ceramide-treated group, and two PK activators-treated groups), and RNAs of the 16 samples were extracted. In detail, cells were lysed by 500 mL of TRIZOL reagent and transported in a 1.5 mL tube. A total of 0.2 mL chloroform was added to the lysates and mixed by the vortex. Then the samples were centrifuged at 12,000 rpm for 15 minutes at 4 °C, and 0.2 mL of the upper aqueous phase was collected into a new 1.5 mL tube. After adding 0.2 mL of 70 % ethanol, the whole sample (0.4 mL) was transported into a RNeasy Mini spin column, which was placed in a 2 mL collection tube. Then the total RNAs were isolated by using Qiagen RNeasy kits following the manufactured protocol.

The quality, quantity, and RNA integrity number (RIN) of the samples were assessed with NanoDrop (Thermo Fisher, USA), and Bioanalyzer (2100, Agilent Technology, USA). For the RNA samples, the ratio of absorbance at 260 and 280 nm, (A260/280) of ~2, (A260/230) of 2.0 - 2.2, and RIN > 8.0 are acceptable. All samples passed the quality control.

The Ilumina Stranded Total RNA Prep, Ligation with Ribo-Zero Plus kit was used for the construction of NGS libraries from the samples with acceptable quality parameters. The amount of input RNA was determined by the quantity of RNA samples. In short, RNA samples were sequenced with 2×100 paired-end reads by the NovaSeq 6000 system. Raw sequencing data (.bcl) was demultiplexed and converted to FASTQ files with DRAGEN Software (v3.9.5). The data was delivered in FASTQ format using Illumina 1.8 quality scores.

### Processing of RNA-seq data

For C2C12 mouse cell line RNA-seq data, the quality of FASTQ files was assessed by FastQC (v0.11.9) software to ensure the quality of every sequenced sample. Kallisto (v0.48.0) [16] was used to quantify the count and transcripts per million (TPM) values of transcripts based on the Mus musculus reference cDNA (version GRCm39, Ensembl release 107). The transcript read counts and TPM values were assembled to gene level using the R package tximport (v1.22.0) [17], with only protein-coding transcripts and genes included. The lowly expressed genes with an average count below 5 were filtered out, resulting in a total of 14,661 genes for the downstream analysis. All the analyzed genes were denoted by Ensembl gene ID and were converted into HGNC (HUGO Gene Nomenclature Committee) gene symbol when needed.

For human skeletal muscle RNA-seq data, the FASTQ files were obtained from the Gene Expression Omnibus (GEO) with the accession of GSE167186. Similarly, Kallisto (v0.48.0) [16] was used for the quantification based on the human reference cDNA (version GRCh38.p13 Ensembl release 108). Based on our pipeline described above, after removing lowly expressed genes, we finally obtained 5,298 genes for the downstream analysis. Human and mouse genes were converted based on homology using the R package biomaRt (v2.50.3) [18],[19] when needed.

### Differential expression analysis

Differential expression analysis was carried out using the R package DESeq2 (v1.34.0) [20] followed by the Benjamini-Hochberg procedure to correct p-values. Adjusted p-value < 0.05 was chosen as the threshold for the significance of differentially expressed genes (DEGs), with log₂ fold change > 0 for up-regulation and log₂ fold change < 0 for down-regulation.

### Principal component analysis

Principal component analysis (PCA) was applied to visualize the distribution samples, which was performed by the R package pcaMethods (v1.86.0) [21] based on the gene expression profiles after variance stabilizing transformation by DESeq2.

### Gene set functional analysis

Gene set overrepresentation analysis (GSOA) was applied to determine whether a list of DEGs of interest was significantly associated with specific Gene Ontology (GO) biological process terms in the mouse cell line dataset, with all 14,661 analysed genes as the background. In addition, gene set enrichment analysis (GSEA) [22] was performed to assess the activation or inhibition of biological pathways in both mouse and human datasets. Based on this method, genes were sorted based on the log₂ fold change in descending order, and the 50 hallmark gene sets from the Molecular Signatures Database (MSigDB) were tested for their significance. Here, a positive normalized enrichment score (NES) indicates that a hallmark gene set is activated in the corresponding gene list ranked by log₂ fold change, and vice versa. The hallmark gene sets were retrieved from the R package msigdbr (v7.5.1). The R package clusterProfiler (v4.2.2) [23],[24] was used for both GSOA and GSEA, with p-values adjusted by the Benjamini-Hochberg procedure. An adjusted p-value < 0.05 was considered significant.

### Gene co-expression network analysis

Weighted Gene Co-expression Network Analysis (WGCNA) [26] was applied to identify the functional modules in which the genes are highly clustered and correlated in the cell line RNA-seq dataset. All 36 cell line samples analysed in this study were included in the co-expression analysis. The top 10,000 genes with the highest standard deviation in WGCNA were kept since the genes with low variation do not contribute to correlation analysis and introduce noise.

A signed co-expression network was constructed where the co-expression between genes was measured by a signed version of Pearson's correlation coefficient (r) defined as (1+r)/2.26. The global network achieved scale-free property (scale-free fitting index = 0.91) when setting the soft-thresholding parameter as 9. The adjacency matrix of all the 10,000 genes was calculated based on the given soft-thresholding parameter and was subsequently converted into a topological overlap matrix (TOM). Hierarchical clustering was performed on the TOM-based distance between genes. The minimal module size was set as 30. Finally, twelve modules (Module-1 to Module-12) were identified after merging similar modules. Functional enrichment analysis for the genes involved in each module was analysed by GSOA using the 10,000 genes in WGCNA as the background. For each module, the eigengene defined as the first principal component of the module genes was calculated to represent the overall gene expression in the module.

### Transcription factor prediction

ChIP-X Enrichment Analysis 3 (ChEA3) web-server tool [27] was used to predict transcription factors (TFs) that drove the transcription of a list of genes of interest based on the enrichment analysis. The ChEA3 database contained a collection of TF-target gene set libraries from multiple sources, including TF-gene co-expression from RNA-seq studies, TF-target associations from ChIP-seq experiments, and TF-gene co-occurrence computed from crowd-submitted gene lists. The total 1,506 genes involved in Module 7 were used as input. The predicted TFs were presented by a mean rank of the enrichment result across all libraries.

### Statistical analysis

Wilcoxon rank-sum test (non-normal distribution) or t-test (normal distribution) were used for statistical testing. Data normality was checked by Shapiro-Wilk test. * p < 0.05. Statistical analysis was conducted in R (v4.3.1).

### Examples - Results

### Sarcopenia characterization

To explore the molecular alterations in sarcopenia patients, publicly available RNA-seq profiling of the lower limb muscles from 24 sarcopenia patients, 29 old healthy subjects, and 19 young healthy subjects (GSE167186 from GEO) was analysed. The average age (with standard deviation) for the sarcopenia patients, old healthy and young healthy subjects was 76.87 ± 7.44, 72.37 ± 7.07, and 22.05 ± 2.95, respectively. The gene log₂ fold change was calculated by differential expression analysis and compared gene expression in old healthy (n = 29) against young healthy (n = 19) subjects, sarcopenia patients (n = 24) against old healthy subjects, and sarcopenia patients against young healthy subjects, respectively. Based on the GSEA of hallmark gene sets extracted from the MSigDB [22], it was found that glycolysis and oxidative phosphorylation were the two most significantly down-regulated pathways (both normalized enrichment score NES < 0, adjusted p-value < 0.05) in sarcopenia patients compared with either old healthy or young healthy subjects, respectively, see Fig.1. In addition, the oxidative phosphorylation pathway was also significantly down-regulated in the old healthy compared to the young healthy subjects, see Fig. 1. These results suggest that the down-regulation of glycolysis is a hallmark of sarcopenia, whereas oxidative phosphorylation is gradually down-regulated in skeletal muscle during aging.

### in vitro sarcopenia model

An *in vitro* sarcopenia model was established by treating C2C12 mouse myoblast cells with 40 µM of C2-ceramide for 1 day (denoted as group Cerid). Then, the RNA-seq data was generated for both treated and control cells to depict the molecular characteristics of the cells. This model has been widely used for studying the disease mechanisms of sarcopenia, but global gene expression profiling has not been conducted. [12]

Cerid samples showed a clear separation from the control samples without ceramide treatment (both n = 4) based on the gene expression profiles, indicating that a distinct molecular phenotype was induced by the treatment, see Fig. 2A. Based on differential expression analysis, we identified 1,073 up-regulated genes and 1,127 down-regulated genes in the Cerid group compared to the control group, see Fig. 2B. Among these DEGs, the most significantly down-regulated genes were found to be expressed in muscle-related cells, including fibroblasts (*Fbln1, Cxcl12, Gas1, Medag*)*,* smooth muscle cells (*Angpt1*)*,* cardiomyocytes, skeletal, and striated muscle (*Fhl1, Tpl1*)*,* see Fig. 2C, with the biological functions significantly associated with extracellular matrix (ECM) organization and muscle contraction. [29],[30] GSOA of the DEGs revealed that the down-regulated genes by C2-ceramide treatment were significantly enriched in DNA damage-associated biological processes, while the up-regulated genes were enriched in wound healing, see Fig. 2D. GSEA confirmed that glycolysis was significantly inhibited, which is consistent with the results of the human sarcopenia RNA-seq analysis, see Fig. 2E. It was further shown that cell cycle (E2F targets), DNA repair, and replication (G2M checkpoint) were significantly inhibited in the disease model, see Fig. 2E. The differential expression information of the genes in the glycolysis pathway extracted from the WikiPathways database were annotated. [31] Hexokinase 2 (*Hk2*), Aldolase A (*Aldoa*)*,* triosephosphate isomerase 1 (*Tpi1*), glycerol-3-phosphate dehydrogenase1 (*Gpd1*), phosphoglycerate kinase1 (*Pgk1*), PKM2, mitochondrial pyruvate carrier 1 (*MpC1*), and pyruvate dehydrogenase kinase 1 (*Pdk1*) were significantly down-regulated by C2-ceramide, while pyruvate dehydrogenase kinase 4 (*Pdk4*) was significantly up-regulated under the treatment of C2-ceramide, see Fig. 3A-B.

By integrating three independent sarcopenia cohorts from different human populations (GEO accession number: GSE111006, GSE111010, and GSE111016) [32], two key gene co-expression clusters that are closely associated with sarcopenia development were identified, with the up-regulated cluster associated with cell migration, proliferation, and differentiation, and down-regulated cluster associated with energy metabolism and mitochondrial dysfunction including oxidative phosphorylation, ATP synthesis, and mitochondrial electron transport functions. Based on GSEA, it was found that the up-regulated cluster (n = 401 genes) in human sarcopenia was also activated in the *in vitro* model (NES = 1.41, adjusted p-value = 1.57E-02). Meanwhile, the down-regulated cluster (n = 298 genes) in human sarcopenia was inhibited in the *in vitro* disease model (NES = -1.52, adjusted p-value = 2.95E-03), suggesting that the established *in vitro* model can well mimic the disease signature.

Phenotypically, as shown by immunofluorescence staining of Myosin-3 - a transcription factor and biomarker of muscle regeneration, [33] much fewer myotubes can be differentiated from myoblasts in the disease model compared to the control group, see Fig. 2F. Taken together, these results highlighted the molecular changes in the sarcopenia disease model, which can also reflect the disease signature in human tissue. In addition, both mouse cell line and human RNA-seq datasets implicated that the glycolysis pathway plays an important role in myoblast differentiation.

### PK activators induce muscle cell differentiation based on the in vitro sarcopenia model

Considering the downregulation of glycolysis in sarcopenia patients, the inventors sought to treat these patients by activating the glycolysis pathway through the use of PK activators. Thus, Mitapivat (MTPV) and TEPP46 were used in the developed sarcopenia model. MTPV has been used to allosterically activate pyruvate kinase [13], whereas TEPP46 has been used to stabilizes the PKM2 tetramer. [14] C2C12 cells were treated with MTPV and TEPP46 together with C2-ceramide for one day (denoted as Cerld+MTPV and Cer1d+TEPP46 groups, respectively), see Fig. 4. Based on the PCA plot, the treatment of the *in vitro* sarcopenia model with the two PK activators resulted in significant effects on cells compared to the Cerid disease model, with a clear separation between PK activator-treated cells and Cerid disease model, see Fig. 5A. Based on differential expression analysis, a total of 167 genes in the Cer1d+MTPV treated cells and 655 genes in the Cer1d+TEPP46 treated cells were significantly dysregulated compared to the Cerid group, see Fig. 5B. The up-regulated genes (observed by GSOA) in the Cer1d+TEPP46 group were significantly associated with muscle cell differentiation and development while the same could not be observed in the Cerid+MTPV group, see Fig. 5C. Similar results were also identified by GSEA, showing that the myogenesis was elevated in both treatments, with a higher significance in the Cer1d+TEPP46 group (adjusted p-value = 4.23E-08), see Fig. 5D.

In addition, we performed immunofluorescence staining and validated that the MTPV and TEPP46 treatments could reactivate myoblast-myotube differentiation, see Fig. 5E, with a much higher amount of myotubes compared to the disease model, see Fig. 2F. To summarize, it was demonstrated that modulation of the glycolysis pathway through the use of MTPV and TEPP46 can reverse the disease progress of sarcopenia *in vitro,* with TEPP46 treatment resulting in the largest effects in the cell experiments.

### PK activators show distinct efficacy in the in vitro sarcopenia model

The efficacy of MTPV and TEPP46 was assessed with pyruvate kinase activity assay, see Fig. 6. Both activators showed an increase in PK activity compared to the control, see Fig. 6A. furthermore, cross-linking was performed to investigate if the treatment with the PK activators could transform PK dimer to active tetramer form. Both MTPV and TEPP46 had the ability to transform the PK dimer form to tetramer form while the cells treated with C2-ceramide showed a low concentration of tetramer form. The effect was more pronounced for the treatment with TEPP46, see Fig. 6B.

RNA-seq data was generated for all cells treated with different PK activators in order to reveal the global biological alterations. Both of the PK activator-treated groups were closely clustered together with the disease model Cerid, which is away from the control group, see Fig. 7A. It was observed that the MPTV treatment led to fewer DEGs compared to TEPP46 treatment, implicating that the effect of MTPV is less pronounced than the effect of TEPP46. This may also explain why treatment with TEPP46 seems more effective than treatment with MTPV based on the *in vitro* sarcopenia model, Fig. 7B.

Based on the GSEA results, the PK activators led to a reduction of the level of the cell cycle (E2F targets), DNA repair, replication (G2M checkpoint), but also significantly up-regulated the myogenesis, Fig. 7C.

Finally, both MTPV and TEPP46 reactivated myoblast-myotube differentiation in the disease model as demonstrated by quantified intensity of Myosin-3 staining intensity, see Fig. 7D.

### Gene co-expression network analysis identified a subnetwork controlling the muscle cell differentiation

Based on the RNA-seq profiling of 12 samples from all treated groups, a co-expression network was constructed, where the inherent gene-gene association can be conserved across all the C2C12 cells. Based on the 10,000 most variable genes, see Fig. 8A, a total of 12 gene co-expression modules with the size varying from 148 (Module-6) to 2,050 (Module 5) were identified, see Fig. 9A-B. These modules reflected a distinct gene expression pattern induced by the treatments of different PK activators, see Fig. 9A.

Among these co-expression modules, Modules 1, 3 and 11 were functionally associated; the same holds for Modules 5, 8, 10 and 12, see Fig. 8B. The genes involved in Modules 1, 3 and 11 were significantly enriched in cell cycle processes, and the genes involved in Modules 5, 10, and 12 were significantly enriched in sensory perception, see Fig. 9B. Importantly, Module-7, consisting of 1,506 genes, was associated with muscle cell development and myotube differentiation, which is the most important module regulating myoblast-myotube differentiation in C2C12 in *vitro* models regardless of the PK activator treatment.

Further, the transcription factors regulating Module-7 were inferred by ChEA3 - a web-based tool for TF enrichment analysis. [27] The top 10 most significant TFs from the top 100 ranked TFs were selected and are shown in Table 1.

**Table 1 shows the top-10 transcription factors (TFs) predicted by ChEA3 for the WGCNA Module-7.**

| **Rank** | **TF** | **Description** |
|---|---|---|
| 1 | MYOG | Myogenin |
| 2 | MYF6 | Myogenic Factor 6 |
| 3 | MYOD1 | Myogenic Differentiation 1 |
| 4 | TBX15 | T-Box Transcription Factor 15 |
| 5 | SIX1 | SIX Homeobox 1 |
| 6 | YBX3 | Y-Box Binding Protein 3 |
| 7 | TEAD4 | TEA Domain Transcription Factor 4 |
| 8 | PRRX2 | Paired Related Homeobox 2 |
| 9 | RXRG | Retinoid X Receptor Gamma |
| 10 | RORC | RAR Related Orphan Receptor C |

Among the top 10 TFs, the three most highly ranked TFs, i.e., MYOG, MYF6, and MYOD1, are all pivotal TFs in muscle cells and myocytes. [29,30] The *Myf6* gene was lowly expressed in the cell lines, but the other two genes (i.e., *Myog* and *Myod1*) were highly expressed (both with an average count > 2,000. We then examined the changes of the genes *Myog* and *Myod1* together with Myh3 (encoding the TF Myosin-3 used for immunofluorescence staining) in the disease model with/without PK activator treatments by comparing them to the control. In the disease model Cerid, we found that all three TFs were significantly down-regulated, Fig. 9C. With the treatment of PK activators, both *Myog* and *Myh3* were not down-regulated in the PK activator-treated groups. In addition, both MTPV and TEPP46 significantly alleviated the down-regulation of *Myod1* in the *in vitro* sarcopenia disease model, see Fig. 9C.

Taken together, by applying gene co-expression network analysis and transcription factor analysis, a muscle cell differentiation-associated gene co-expression module driven by transcription factor *Myog* and *Myod1* was identified. Furthermore, it was observed that the down-regulation of these TFs in the disease model Cerid can be alleviated by the treatment with PK activators.

### REFERENCES

[1] Argiles JM, Campos N, Lopez-Pedrosa JM, Rueda R, Rodriguez-Manas L. Skeletal Muscle Regulates Metabolism via Interorgan Crosstalk: Roles in Health and Disease. J Am Med Dir Assoc 2016; 17(9): 789-96.
[2] Frontera WR, Ochala J. Skeletal Muscle: A Brief Review of Structure and Function. Calcified Tissue International 2015; 96(3): 183-95.
[3] Wolfe RR. The underappreciated role of muscle in health and disease. The American Journal of Clinical Nutrition 2006; 84(3): 475-82.
[4] Buckingham M, Rigby PW. Gene regulatory networks and transcriptional mechanisms that control myogenesis. Dev Cell 2014; 28(3): 225-38.
[5] Fulco M, Cen Y, Zhao P, et al. Glucose Restriction Inhibits Skeletal Myoblast Differentiation by Activating SIRT1 through AMPK-Mediated Regulation of Nampt. Developmental Cell 2008; 14(5): 661-73.
[6] Luo W, Ai L, Wang B-f, Zhou Y. High glucose inhibits myogenesis and induces insulin resistance by down-regulating AKT signaling. Biomedicine & Pharmacotherapy 2019; 120: 109498.
[7] Valentini G, Chiarelli L, Fortin R, Speranza ML, Galizzi A, Mattevi A. The Allosteric Regulation of Pyruvate Kinase: A SITE-DIRECTED MUTAGENESIS STUDY*. Journal of Biological Chemistry 2000; 275(24): 18145-52.
[8] Noguchi T, Inoue H, Tanaka T. The M1- and M2-type isozymes of rat pyruvate kinase are produced from the same gene by alternative RNA splicing. Journal of Biological Chemistry 1986; 261(29): 13807-12.
[9] Zhang Z, Deng X, Liu Y, Liu Y, Sun L, Chen F. PKM2, function and expression and regulation. Cell & Bioscience 2019; 9(1): 52.
[10] Blum JE, Gheller BJ, Benvie A, et al. Pyruvate Kinase M2 Supports Muscle Progenitor Cell Proliferation but Is Dispensable for Skeletal Muscle Regeneration after Injury. The Journal of Nutrition 2021; 151(11): 3313-28.
[11] Kim M, Zhang Y, Birchmeier C. Fructose 1,6-bisphosphate sensing by pyruvate kinase isozymes M2 (PKM2) controls MyoD stability and myogenic differentiation. bioRxiv 2020: 2020.12.22.424062.
[12] Jadhav KS, Dungan CM, Williamson DL. Metformin limits ceramide-induced senescence in C2C12 myoblasts. Mechanisms of Ageing and Development 2013; 134(11): 548-59.
[13] Al-Samkari H, van Beers EJ. Mitapivat, a novel pyruvate kinase activator, for the treatment of hereditary hemolytic anemias. Therapeutic Advances in Hematology 2021; 12: 20406207211066070.
[14] Anastasiou D, Yu Y, Israelsen WJ, et al. Pyruvate kinase M2 activators promote tetramer formation and suppress tumorigenesis. Nature Chemical Biology 2012; 8(10): 839-47.
[15] Schneider CA, Rasband WS, Eliceiri KW. NIH Image to ImageJ: 25 years of image analysis. Nature Methods 2012; 9(7): 671-5.
[16] Bray NL, Pimentel H, Melsted P, Pachter L. Near-optimal probabilistic RNA-seq quantification. Nature Biotechnology 2016; 34(5): 525-7.
[17] Soneson C, Love M, Robinson M. Differential analyses for RNA-seq: transcript-level estimates improve gene-level inferences. F1000Research 2016; 4(1521).
[18] Durinck S, Spellman PT, Birney E, Huber W. Mapping identifiers for the integration of genomic datasets with the R/Bioconductor package biomaRt. Nature Protocols 2009; 4(8): 1184-91.
[19] Durinck S, Moreau Y, Kasprzyk A, et al. BioMart and Bioconductor: a powerful link between biological databases and microarray data analysis. Bioinformatics 2005; 21(16): 3439-40.
[20] Love MI, Huber W, Anders S. Moderated estimation of fold change and dispersion for RNA-seq data with DESeq2. Genome Biology 2014; 15(12): 550.
[21] Stacklies W, Redestig H, Scholz M, Walther D, Selbig J. pcaMethods-a bioconductor package providing PCA methods for incomplete data. Bioinformatics 2007; 23(9): 1164-7.
[22] Subramanian A, Tamayo P, Mootha VK, et al. Gene set enrichment analysis: A knowledge-based approach for interpreting genome-wide expression profiles. Proceedings of the National Academy of Sciences 2005; 102(43): 15545-50.
[23] Yu G, Wang L-G, Han Y, He Q-Y. clusterProfiler: an R Package for Comparing Biological Themes Among Gene Clusters. OMICS: A Journal of Integrative Biology 2012; 16(5): 284-7.
[24] Wu T, Hu E, Xu S, et al. clusterProfiler 4.0: A universal enrichment tool for interpreting omics data. The Innovation 2021; 2(3): 100141.
[25] Yu G, Li F, Qin Y, Bo X, Wu Y, Wang S. GOSemSim: an R package for measuring semantic similarity among GO terms and gene products. Bioinformatics 2010; 26(7): 976-8.
[26] Langfelder P, Horvath S. WGCNA: an R package for weighted correlation network analysis. BMC Bioinformatics 2008; 9(1): 559.
[27] Keenan AB, Torre D, Lachmann A, et al. ChEA3: transcription factor enrichment analysis by orthogonal omics integration. Nucleic Acids Research 2019; 47(W1): W212-W24.
[28] Roth L, Van Dam D, Van der Donckt C, et al. Impaired gait pattern as a sensitive tool to assess hypoxic brain damage in a novel mouse model of atherosclerotic plaque rupture. Physiology & Behavior 2015; 139: 397-402.
[29] Uhlén M, Fagerberg L, Hallström BM, et al. Tissue-based map of the human proteome. Science 2015; 347(6220): 1260419.
[30] Karlsson M, Zhang C, Méar L, et al. A single-cell type transcriptomics map of human tissues. Science Advances 2021; 7(31): eabh2169.
[31] Martens M, Ammar A, Riutta A, et al. WikiPathways: connecting communities. Nucleic Acids Research 2020; 49(D1): D613-D21.
[32] Migliavacca E, Tay SKH, Patel HP, et al. Mitochondrial oxidative capacity and NAD+ biosynthesis are reduced in human sarcopenia across ethnicities. Nature Communications 2019; 10(1): 5808.
[33] Schiaffino S, Rossi AC, Smerdu V, Leinwand LA, Reggiani C. Developmental myosins: expression patterns and functional significance. Skeletal Muscle 2015; 5(1): 22.
[34] Christian CJ, Benian GM. Animal models of sarcopenia. Aging Cell 2020; 19(10): e13223.
[35] Mankhong S, Kim S, Moon S, Kwak H-B, Park D-H, Kang J-H. Experimental Models of Sarcopenia: Bridging Molecular Mechanism and Therapeutic Strategy. Cells 2020; 9(6): 1385.
[36] Kim JW, Kim R, Choi H, Lee S-J, Bae G-U. Understanding of sarcopenia: from definition to therapeutic strategies. Archives of Pharmacal Research 2021; 44(9): 876-89.
[37] Yanay N, Rabie M, Nevo Y. Impaired Regeneration in Dystrophic Muscle-New Target for Therapy. Frontiers in Molecular Neuroscience 2020; 13.
[38] Ciscato F, Ferrone L, Masgras I, Laquatra C, Rasola A. Hexokinase 2 in Cancer: A Prima Donna Playing Multiple Characters. International Journal of Molecular Sciences 2021; 22(9): 4716.
[39] Casciola-Rosen L, Hall JC, Mammen AL, Christopher-Stine L, Rosen A. Isolated elevation of aldolase in the serum of myositis patients: a potential biomarker of damaged early regenerating muscle cells. Clinical and experimental rheumatology 2012; 30(4): 548-53.
[40] Pin F, Novinger LJ, Huot JR, et al. PDK4 drives metabolic alterations and muscle atrophy in cancer cachexia. The FASEB Journal 2019; 33(6): 7778-90.
[41] Verbrugge SAJ, Gehlert S, Stadhouders LEM, et al. PKM2 Determines Myofiber Hypertrophy In Vitro and Increases in Response to Resistance Exercise in Human Skeletal Muscle. International Journal of Molecular Sciences 2020; 21(19): 7062.
[42] Zhao X, Tan F, Cao X, et al. PKM2-dependent glycolysis promotes the proliferation and migration of vascular smooth muscle cells during atherosclerosis. Acta Biochimica et Biophysica Sinica 2019; 52(1672-9145): 9.
[43] Ferri E, Marzetti E, Calvani R, Picca A, Cesari M, Arosio B. Role of Age-Related Mitochondrial Dysfunction in Sarcopenia. International Journal of Molecular Sciences 2020; 21(15): 5236.
[44] Wiedmer P, Jung T, Castro JP, et al. Sarcopenia - Molecular mechanisms and open questions. Ageing Research Reviews 2021; 65: 101200.
[45] Ribeiro AJS, Yang X, Patel V, Madabushi R, Strauss DG. Liver Microphysiological Systems for Predicting and Evaluating Drug Effects. Clinical Pharmacology & Therapeutics 2019; 106(1): 139-47.
[46] Morley JE. Pharmacologic Options for the Treatment of Sarcopenia. Calcified Tissue International 2016; 98(4): 319-33.
[47] Kwak JY, Kwon K-S. Pharmacological Interventions for Treatment of Sarcopenia: Current Status of Drug Development for Sarcopenia. Ann Geriatr Med Res 2019; 23(3): 98-104.

## Claims

1. A compound or a pharmaceutically acceptable salt or prodrug thereof for use in a method of treatment of human sarcopenia, wherein the compound is Mitapivat or TEPP46.

2. The compound for use according to claim 1, wherein the compound is Mitapivat.

3. The compound for use according to claim 1, wherein the compound is TEPP46.
